# EUROPEAN PATENT APPLICATION

(11) **EP 4 122 491 A1**
(43) Date of publication of application: **25.01.2023**
(21) Application number: 21187253.6
(22) Date of filing: 22.07.2021
(51) Int. Cl.: A61K 39/04, A61P 13/10, A61P 35/00

(54) **RECOMBINANT MICROBACTERIUM AS AN IMMUNOTHERAPEUTIC AGENT FOR THE SECOND-LINE THERAPY OF BLADDER CARCINOMA**

(71) Applicant: Vakzine Projekt Management GmbH, 30625 Hannover (DE); Serum Institute Of India Private Limited, Pune, Maharashtra 411 028 (IN)
(72) Inventor: GRODE, Leander, 38110 Braunschweig (DE)
(74) Representative: Weiss, Wolfgang

(57) **Abstract**

The invention relates to a recombinant Mycobacterium cell for use as an immunotherapeutic agent in the treatment of bladder carcinoma, particularly in the second-line treatment of non-muscle-invasive bladder carcinoma.

## Description

The invention relates to a recombinant Mycobacterium cell for use as an immunotherapeutic agent in the therapy of bladder carcinoma, particularly in the second-line therapy of non-muscle-invasive bladder carcinoma.

Urothelial bladder carcinoma is the 5th most common cancer. In the United States, about 75.000 new cases are diagnosed each year 4.5% of all new cancers, and approximately 15.600 deaths are expected. In Germany, about 16.000 new cases are diagnosed each year. Because a recurrence of disease is likely in bladder carcinoma, patients must undergo surveillance for an extended period.

Most bladder carcinomas begin in transitional epithelial cells that make up the inner lining of the bladder. As these tumors grow, they can invade the surrounding connective tissue and muscle. In advanced disease, tumors spread beyond the bladder to nearby lymph nodes or pelvic organs or metastasize to more distant organs such as lung, liver and bone.

The overall 5-year survival rate for bladder carcinoma is 77%, and this rate has not changed significantly over the last 10 years. When considered by stage, the 5-year relative survival rates for patients with tumors restricted to the inner layer of the bladder are 96% and 69%, respectively. The rates drop to 34% for those with disease that has spread locally beyond the bladder and to 6% with distant metastases.

In the treatment of bladder carcinoma, tumor recurrence is a major concern, even for patients with low-grade disease and requires extensive follow-up. Better treatments, such as novel immunotherapies, might reduce recurrence rates and improve the survival of patients with bladder carcinoma.

For patients with non-muscle invasive bladder carcinoma, treatment usually involves a surgical removal of the tumor followed by chemotherapy, usually mitomycin C, within the bladder (so-called intravesical chemotherapy). After recovering from surgery, patients with a lower risk of disease progression may undergo surveillance or additional intravesical chemotherapy. Patients with moderate-to-high-grade disease often receive intravesical immunotherapy with an attenuated live bacterium Bacillus Calmette Guerin (BCG). BCG was the first FDA-approved immunotherapy and helps reduce the risk of bladder carcinoma recurrence by stimulating an immune response that targets the bacteria as well as any bladder carcinoma cells. Despite the proven efficacy of BCG treatment, about 35 to 45% of patients experience disease recurrence by 5 years and in about 10 to 13% of patients the disease progresses of (Oddens et al., Eur Urol 63 (2013, 462-472).

Recurrence and progression to muscle-invasive disease may lead to additional chemotherapeutical, surgical and radio-oncological interventions including cisplatin-based chemotherapy, transurethral resection of the bladder (TURB), cystectomy and chemo-radiotherapy. Recurrent bladder carcinoma may be treated with combination therapy regimens, including gemcitabine plus cisplatin or methotrexate, vinblastine, doxorubicin plus cisplatin.

An alternative option for recurrent patients is a re-treatment with BCG (Yates et al, Eur Urol 62 (2012), 1088-1096). The earlier the BCG failure, the more probable is the failure of a second BCG cycle (Gallagher et al., Urology 71 (2008), 297-301).

A second-line BCG immunotherapy after initial BCG failure in non-muscle-invasive bladder cancer, however, was found to be not effective (Di Lorenzo et al.,Cancer 2010, doi: 10.1002/cncr.24914). 87.5% patients failed to respond to BCG re-induction at one year. 37.5% of the patients had to undergo cystectomy and 40% underwent radiation therapy plus systemic chemotherapy after one year. The poor outcome in these patients failing to respond to BCG therapy reflects the unmet medical need for improved bladder-sparing treatments after BCG or other intravesical treatment failure. Thus, better treatment options are not only needed for a first line therapy but also for patients with recurrence after a first course of standard BCG therapy.

Thus, it was an object of the present invention to provide an improved treatment for patients failing to respond to first line BCG therapy as these patients are at high risk of cancer progression. An improved treatment of these high-risk patients will increase bladder preservation rates and consequently improve quality of life and decrease health costs.

A recombinant BCG strain expressing a phagolysosomal escape domain is described in WO 99/101496, the content of which is herein incorporated by reference. The phagolysosomal escape domain enables the strain to escape from the phagosome of infected host cells by perforating the membrane of the phagosome. In order to provide an acidic phagosomal pH for optimal phagolysosomal escape activity, a urease-deficient recombinant strain was developed. This strain is disclosed in WO 2004/094469, the content of which is herein incorporated.

WO 2012/085101, the content of which is herein incorporated, discloses that a recombinant BCG strain expressing membrane-perforating listeriolysin (Hly) of *Listeria monocytogenes* and devoid of urease C induces superior protection against aerogenic challenge with *Mycobacterium tuberculosis (Mtb)* as compared to parental BCG in a preclinical model. Further, it is shown that both the recombinant and the parenteral strain induce marked Th1 immune responses, whilst only the recombinant BCG strain elicits are profound Th17 response in addition.

WO 2016/177717 discloses that a recombinant urease-deficient and listeriolysin-expressing recombinant BCG strain induces a superior immune response compared to the parenteral BCG in an animal model. Further, the start of a human clinical phase I/II trial using recombinant BCG as an immunotherapeutic agent in patients after a first standard BCG therapy is reported.

Despite the discouraging results of Di Lorenzo et al., supra, the present inventors have surprisingly observed a high overall survival and a low disease recurrence rate even 3 years after the start of therapy using a urease-deficient and listeriolysin-expressing recombinant BCG as a second-line BCG immunotherapy after initial BCG failure in non-muscle-invasive bladder cancer.

A first aspect of the present invention relates to a recombinant Mycobacterium cell, which comprises a recombinant nucleic acid molecule encoding a fusion polypeptide comprising:
(a) a domain capable of eliciting an immune response, and
(b) a phagolysosomal escape domain
for use in a method, wherein the recombinant Mycobacterium cell is administered as an immunotherapeutic agent to a subject suffering from bladder carcinoma, particularly from non-muscle-invasive bladder carcinoma as a second-line therapy.

Accordingly, the present invention further relates to a method for the immunotherapeutic treatment of bladder carcinoma, particularly of non-muscle-invasive bladder carcinoma in a subject, particularly in a human subject, comprising administering to said subject as a second-line therapy a recombinant Mycobacterium cell, which comprises a recombinant nucleic acid molecule encoding a fusion polypeptide comprising:
(a) a domain capable of eliciting an immune response, and
(b) a phagolysosomal escape domain.

A second aspect of the present invention relates to a recombinant Mycobacterium cell, which comprises a recombinant nucleic acid molecule encoding a fusion polypeptide comprising:
(a) a domain capable of eliciting an immune response, and
(b) a phagolysosomal escape domain
for use in a method, wherein the recombinant Mycobacterium cell is administered as an immunotherapeutic agent to a subject suffering from bladder carcinoma, particularly from non-muscle-invasive bladder carcinoma, wherein disease recurrence is inhibited for a time period of at least 1 year, particularly for a time period of at least 2 years or of at least 3 years.

Accordingly, the present invention further relates to a method for the immunotherapeutic treatment of bladder carcinoma, particularly of non-muscle-invasive bladder carcinoma in a subject, particularly in a human subject, comprising administering to said subject a recombinant Mycobacterium cell, which comprises a recombinant nucleic acid molecule encoding a fusion polypeptide comprising:
(a) a domain capable of eliciting an immune response, and
(b) a phagolysosomal escape domain,
wherein disease recurrence is inhibited for a time period of at least 1 year, particularly for a time period of at least 2 years or of at least 3 years.

A third aspect of the present invention relates to a recombinant Mycobacterium cell, which comprises a recombinant nucleic acid molecule encoding a fusion polypeptide comprising:
(a) a domain capable of eliciting an immune response, and
(b) a phagolysosomal escape domain
for use in a method, wherein the recombinant Mycobacterium cell is administered as an immunotherapeutic agent to a subject suffering from bladder carcinoma, particularly from non-muscle-invasive bladder carcinoma, wherein overall survival is increased in a time period of at least 1 year, particularly in a time period of at least 2 years or of at least 3 years.

Accordingly, the present invention further relates to a method for the immunotherapeutic treatment of bladder carcinoma, particularly of non-muscle-invasive bladder carcinoma in a subject, particularly in a human subject, comprising administering to said subject a recombinant Mycobacterium cell, which comprises a recombinant nucleic acid molecule encoding a fusion polypeptide comprising:
(a) a domain capable of eliciting an immune response, and
(b) a phagolysosomal escape domain,
wherein overall survival is increased in a time period of at least 1 year, particularly in a time period of at least 2 years or of at least 3 years.

A fourth aspect of the present invention relates to a recombinant Mycobacterium cell, which comprises a recombinant nucleic acid molecule encoding a fusion polypeptide comprising:
(a) a domain capable of eliciting an immune response, and
(b) a phagolysosomal escape domain
for use in a method for improving the life quality of a subject suffering from bladder carcinoma, particularly from non-muscle-invasive bladder carcinoma, wherein the recombinant Mycobacterium cell is administered as an immunotherapeutic agent thereby reducing or avoiding cystectomy in said subject.

Accordingly, the present invention further relates to a method for improving the life quality of a subject suffering from bladder carcinoma, particularly from non-muscle-invasive bladder carcinoma, particularly in a human subject, comprising administering to said subject a recombinant Mycobacterium cell, which comprises a recombinant nucleic acid molecule encoding a fusion polypeptide comprising:
(a) a domain capable of eliciting an immune response, and
(b) a phagolysosomal escape domain,
and avoiding or reducing the need of cystectomy in said subject.

It should be noted, that two or more of the features of the above-indicated aspects may be combined with each other.

Thus, the present invention relates to a second-line therapy in combination with at least one of:
- an inhibition of disease recurrence for a time period of at least 1 year, particularly for a time period of at least 2 years or of at least 3 years,
- an increase of overall survival to a time period of at least 1 year, particularly for a time period of at least 2 years or of at least 3 years, and
- an improvement of life quality comprising reducing or the need of cystectomy.

Further, the present invention relates to an improvement of life quality comprising reducing or the need of cystectomy in combination with at least one of:
- a second-line therapy for a subject having a disease recurrence and/or disease progression after a failed first-line therapy,
- an inhibition of disease recurrence for a time period of at least 1 year, particularly for a time period of at least 2 years or of at least 3 years,
- an increase of overall survival to a time period of at least 1 year, and particularly for a time period of at least 2 years or of at least 3 years.

In a particular embodiment, the present invention relates to a second-line therapy in combination with:
- an inhibition of disease recurrence for a time period of at least 1 year, particularly for a time period of at least 2 years or of at least 3 years,
- an increase of overall survival to a time period of at least 1 year, particularly for a time period of at least 2 years or of at least 3 years, and optionally
- an improvement of life quality comprising reducing or the need of cystectomy.

The present inventors have found that a second-line immunotherapy comprising administration of a recombinant Mycobacterium cell as described herein was highly effective in reducing disease occurrence and increasing overall survival. In this context, the term "second-line immunotherapy" refers to the treatment of a subject, particularly a human subject, after failure of a first-line therapy. The term "failure" particularly means disease recurrence and/or disease progression including progression of the primary tumor and/or formation of metastases after first-line therapy. More particularly, the term "failure" means disease recurrence. The term "first-line therapy" particularly refers to a first-line immunotherapy, and more particularly to a first-line immunotherapy comprising administration of BCG without a phagolysosomal escape domain as described herein, such as previously approved standard BCG as described in Rentsch, C A, Eur Urol 2014, PMID: 24674149. The term "second-line immunotherapy" comprises administration of a recombinant Mycobacterium cell as described herein after a single "first-line therapy" cycle or after multiple, e.g. 2, 3 or more "first-line therapy" cycles, particularly a single cycle or multiple cycles comprising administration of BCG without a phagolysosomal escape domain.

The subject to be treated according to the present invention is suffering from bladder carcinoma, particularly non-muscle invasive bladder carcinoma (NMIBC) including carcinoma in situ (CIS). In particular embodiments, the subject is suffering from recurrent NMBIC. In some embodiments, the subject has a recurrent high-grade NMIBC for progression (e.g. score 7-23) based on the European Organization for Research and Treatment. In further embodiments, the subject has undergone to a transurethral resection of the bladder tumor (TURBT) before therapy start. In some embodiments, the subject has a tumor-free bladder state at therapy start, e.g. as confirmed by transurethral resection of the bladder (TURB) and biopsy.

In particular embodiments, the subject is a patient with a high grade NMIBC tumor present at 6 months or both at 3 months and 6 months after start of a previous cycle of standard BCG therapy, and worsening of the disease under standard BCG therapy including at least one of higher number of recurrences, higher tumor category or higher grade, and appearances of CIS, including low grade tumors, optionally in spite of an initial response.

Administration of a recombinant Mycobacterium cell as described herein as a second-line therapy is highly effective in reducing disease occurrence for an extended time period, e.g. a time period of at least 1 year, particularly for a time period of at least 2 years or of at least 3 years. 3 years after treatment start. At present, the median time of disease recurrence is already 1.3 years and will probably increase further. At this time, no tumor recurrence was observed in about 49% of the patients.

Thus, the therapy of the present invention provides a disease free time period of at least 1 year, particularly of at least 2 years or of at least 3 years for at least 30% of the patients and particularly for at least 45% of the patients.

Notably, in the time period between 2 and 3 years after treatment start, no new disease recurrence was observed. Thus, the treatment is highly efficient in stopping disease recurrence in a sub-group of patients, who did not suffer from disease recurrence in a time period up to 2 years after treatment start.

In contrast thereto, administration of a standard BCG as a second-line therapy was not effective at all as described by Di Lorenzo et al. supra, since 2 years after treatment start in only less than 5% of the patients no tumor recurrence was observed.

This high clinical efficiency of the therapy of the present invention was found without the occurrence of clinically significant adverse events. Thus, the treatment translates into an overall survival benefit for the patients.

Thus, administration of a recombinant Mycobacterium cell as described herein is highly effective in increasing the overall survival in an extended time period, e.g. a time period of at least 1 year, particularly in a time period of at least 2 years or of at least 3 years. 3 years after start of the treatment, the overall survival rate was between 70% and 80%.

In particular embodiments, the therapy of the invention is suitable in avoiding cystectomy in a large number of patients resulting in a high improvement of life quality. For example, cystectomy may be avoided for a time period of at least a 1 year, particularly for a time period of at least 2 years or of at least 3 years in at least 30% of the patients and particularly for at least 45% of the patients.

The immunotherapeutic agent is a live recombinant Mycobacterium cell, which comprises a recombinant nucleic acid molecule encoding a fusion polypeptide comprising (a) a domain capable of eliciting an immune response and (b) a phagolysosomal escape domain. The domain capable of eliciting an immune response is preferably an immunogenic peptide or polypeptide from a pathogen or an immunogenic fragment thereof.

The Mycobacterium cell is preferably an *M. bovis* cell, an *M. tuberculosis* cell, particularly an attenuated *M. tuberculosis* cell or other Mycobacteria, e.g. *M. microti, M. smegmatis, M. canettii, M. marinum* or *M. fortuitum.* More preferably, the cell is an attenuated recombinant *M. bovis* (BCG) cell, particularly an *M. bovis* BCG cell, more particularly a recombinant *M. bovis* BCG cell from strain Danish subtype Prague (Brosch et al., Proc. Natl. Acad. Sci. USA, 104 (2007), 5396-5601). In an especially preferred embodiment, the Mycobacterium cell is recombinant urease-deficient. In an especially preferred embodiment the ureC sequence of the Mycobacterium cell is inactivated (ΔUrec), e.g. by constructing a suicide vector containing a ureC gene disrupted by a selection marker gene, e.g. the hygromycin gene, transforming the target cell with the vector and screening for selection marker-positive cells having a urease negative phenotype. In an even more preferred embodiment, the selection marker gene, i.e. the hygromycin gene, is subsequently inactivated. In this embodiment, the cell is a selection marker-free recombinant Mycobacterium cell. Most preferably, the cell is selection marker-free recombinant BCG strain Danish subtype Prague characterized as recombinant BCG ΔUrec::Hly+.

The domain capable of eliciting an immune response is preferably selected from immunogenic peptides or polypeptides from *M. bovis, M. tuberculosis* or *M. leprae* or from immunogenic fragments thereof having a length of at least 6, preferably at least 8 amino acids, more preferably at least 9 amino acids and e.g. up to 20 amino acids. Specific examples for suitable antigens are Ag85B (p30) from *M. tuberculosis,* Ag85B (a-antigen) from *M. bovis* BCG, Ag85A from *M. tuberculosis* and ESAT-6 from *M. tuberculosis* and fragments thereof. In other embodiments, the domain capable of eliciting an immune response is selected from non-Mycobacterium polypeptides.

More preferably, the immunogenic domain is derived from the antigen Ag85B. Most preferably, the immunogenic domain comprises the sequence from aa. 41 to aa.51 in SEQ ID No.2.

The recombinant nucleic acid molecule further comprises a phagolysosomal escape domain, i.e. a polypeptide domain which provides for an escape of the fusion polypeptide from the phagolysosome into the cytosol of mammalian cells. Preferably, the phagolysosomal escape domain is a Listeria phagolysosomal escape domain, which is described in US 5,733,151, herein incorporated by reference. More preferably, the phagolysosomal escape domain is derived from the listeriolysin gene (Hly) of L. monocytogenes. Most preferably, the phagolysosomal domain is encoded by a nucleic acid molecule selected from: (a) a nucleotide sequence comprising nucleotides 211 - 1722 as shown in SEQ ID No.1, (b) a nucleotide sequence which encodes the same amino acid sequence as the sequence from (a), and (c) a nucleotide sequence hybridizing under stringent conditions with the sequence from (a) or (b).

Apart from the nucleotide sequence depicted in SEQ ID No.1 the present invention also comprises nucleic acid sequences hybridizing therewith. In the present invention, the term "hybridization" is used as defined in Sambrook et al. (Molecular Cloning. A laboratory manual, Cold Spring Harbor Laboratory Press (1989), 1.101-1.104). In accordance with the present invention the term "hybridization" is used if a positive hybridization signal can still be observed after washing for one hour with 1 X SSC and 0.1 % SDS at 55°C, preferably at 62° C and more preferably at 68°C, particularly for 1 hour in 0.2 X SSC and 0.1 % SDS at 55°C, preferably at 62°C and more preferably at 68°C. A sequence hybridizing with a nucleotide sequence as per SEQ ID No.1 under such washing conditions is a phagolysosomal escape domain encoding nucleotide sequence preferred by the subject invention.

A nucleotide sequence encoding a phagolysosomal escape domain as described above may be directly obtained from a Listeria organism or from any recombinant source e.g. a recombinant *E.coli* cell containing the corresponding Listeria nucleic acid molecule or a variant thereof as described above.

Preferably, the recombinant nucleic acid molecule encoding for a fusion polypeptide contains a signal peptide encoding sequence. More preferably, the signal sequence is a signal sequence active in Mycobacteria, preferably in *M.bovis,* e.g. a native *M.bovis* signal sequence. A preferred example of a suitable signal sequence is the nucleotide sequence coding for the Ag85B signal peptide, which is depicted in SEQ ID No.1 from nucleotide 1 to 120.

Further, it is preferred that a peptide linker be provided between the immunogenic domain and the phagolysosomal escape domain. Preferably, said peptide linker has a length of from 5 to 50 amino acids. More preferably, a sequence encoding a linker as shown in SEQ ID No.1 from nucleotide 154 to 210 or a sequence corresponding thereto as regards the degeneration of the genetic code.

The nucleic acid may be located on a recombinant vector. Preferably, the recombinant vector is a prokaryotic vector, i.e. a vector containing elements for replication or/and genomic integration in prokaryotic cells. Preferably, the recombinant vector carries the nucleic acid molecule of the present invention operatively linked with an expression control sequence. The expression control sequence is preferably an expression control sequence active in Mycobacteria, particularly in *M.bovis.* The vector can be an extrachromosomal vector or a vector suitable for integration into the chromosome. Examples of such vectors are known to the man skilled in the art and, for instance, given in Sambrook *et al.* supra.

The immunotherapeutic agent of the present invention is suitable for the treatment of bladder carcinoma, e.g., non-invasive bladder carcinoma, e.g. non-invasive papillary carcinoma *in situ* (Tₐ), non-invasive carcinoma in situ (T_{cis}), tumor invading subepithelial connective tissue (T₁), tumor invading superficial muscle (inner half) (T₂ₐ), tumor invading deep muscle (outer half) (T2b), tumor invading perivesical tissue (T3 including T3a and T3b), tumor invading prostate, uterus or vagina (T₄ₐ), and tumor invading pelvic wall or abdominal wall (T_{4b}). Particularly, the tumor is a superficial tumor or carcinoma in situ (T_{cis}), non-invasive papillary carcinoma (Tₐ), or a tumor invading subepithelial connective tissue (T₁). The immunotherapeutic treatment is suitable in the treatment of primary bladder carcinoma and/or in the treatment of recurring bladder carcinoma.

The immunotherapeutic agent is locally administered to the tumor site, i.e., to the site of a primary tumor before surgery or after surgery and optionally after chemotherapy. For the treatment of urothelial bladder carcinoma, the agent is preferably administered by vesicular instillation into the urinary bladder.

The immunotherapeutic agent is administered to the subject to be treated in an effective dose. For a human subject, the dose for an administration may be about 10⁶ to 10¹⁰ viable units (CFU), e.g. about 10⁷ to 10⁹ or 10⁸ to 10⁹ viable units. Preferably, the dose for an administration is about 10⁷ to 2 x 10⁸ viable units (CFU). Preferably, the immunotherapeutic agent is administered several times, e.g. at least 3 times or at least 5 times up to 30 times, particularly about 15 times, at predetermined times during the treatment.

The immunotherapeutic agent is usually provided as a pharmaceutical preparation, which comprises the recombinant Mycobacterial cell in solid form, e.g., a lyophilized or cryoconserved preparation, which is reconstituted with a suitable liquid carrier before use. Alternatively, the preparation may be provided in liquid form, e.g., as suspension.

In one embodiment, the immunotherapeutic agent of the invention is administered for the treatment of carcinoma *in situ.* A standard schedule may comprise weekly administration of the agent for at least 4, e.g., 4, 5, 6, 7 or 8 weeks as an induction therapy. The induction therapy should not start until 2-3 weeks after primary tumor surgery. After a treatment-free interval of, e.g., 4 weeks, administration may continue using maintenance therapy for at least 6 months or at least 1 year.

In a further embodiment, the immunotherapeutic agent is administered in an induction therapy in the prophylactic treatment of tumor recurrence. In this embodiment, therapy may start about 2-3 weeks after biopsy of the tumor site and be repeated, e.g., at weekly intervals for at least 4, e.g., 4, 5, 6, 7 or 8 weeks. In intermediate and high-risk tumors this may be followed by maintenance therapy.

Maintenance therapy may comprise long-term therapy, e.g., 6, 9 or 12 months therapy or even longer with treatments at monthly intervals. Alternatively, maintenance therapy may comprise 2, 3 or 4 administrations at weekly intervals, at month 3, 6, 12, 18, 24, 30 and 36.

In a particular embodiment, the immunotherapeutic agent, particularly recombinant BCG ΔUrec::Hly+, is used for the treatment of non-muscle invasive bladder cancer in patients with recurrence after standard BCG therapy. The immunotherapeutic agent is administered into the bladder according to a schedule involving weekly instillations during an induction phase with e.g. 6 weekly instillations, a first maintenance phase after about 3 months with e.g. 3 weekly instillations, a second maintenance phase after about 6 months with e.g. 3 instillations and a third maintenance phase after about 12 months with e.g. 3 instillations.

The administration as immunotherapeutic agent of the recombinant Mycobacterium cell as described above may be combined with further antitumor therapy, e.g., radiation and/or chemotherapy. Further, the immunotherapy as described above may be combined with a non-tumor site specific administration of the recombinant Mycobacterium cell in order to provide a general stimulation of the immune system. This non-site specific administration may be effected as described in WO 2012/085101, e.g. before surgery of the primary tumor. In this case, the agent is preferably administered to a human subject in a dose of about 1-10 x 10⁵, preferably about 2-8 x 10⁵ cells. The agent is preferably administered as a single dose, e.g., by injection. Subcutaneous injection is preferred. Further, it is preferred to administer the agent without adjuvant.

Further, the invention is described in more detail by the following Figures and Example 1.

The immunotherapeutic agent "*rBCG*" used in this example is recombinant *M*. *bovis* (BCG) Danish subtype Prague with an inactivated ureC sequence (ΔUrec) and without functional selection marker gene which expresses an Ag85B/Hly fusion protein as shown in SEQ ID No.2 (Hly+).

### Example 1 Phase I/II open label clinical trial assessing safety and efficacy of intravesical instillation of recombinant BCG (rBCG) in human patients with recurrent non-muscle invasive bladder cancer after standard BCG therapy

### 1.1 Phase I

Phase I was conducted to determine safety, tolerability and the recommended phase II dose of intravesical rBCG instillations in patients with recurrence of non-muscle-invasive bladder cancer after TURB and standard BCG therapy.

### 1.2 Phase II

Phase II was conducted to investigate the efficacy, safety, tolerability and immunogenicity of intravesical rBCG instillations in patients with recurrence of non-muscle-invasive bladder cancer after TURB in standard BCG therapy.

### 1.3 Clinical protocol

rBCG was administered into bladder in 15 weekly instillations (induction phase: instillations 1-6; maintenance 3 months: instillations 7-9; maintenance 6 months: instillations 10-12, maintenance 12 months: instillations 13-15).

The primary endpoint of the phase I was dose limiting toxicity (DLT) of intravesical rBCG instillations in patients with recurrence after standard BCG therapy in non-muscle invasive bladder cancer (NMIBC). The DLT period corresponds to 3 instillations plus 1 week and covers acute toxicities induced by treatment. Patients were treated in two cohorts of three, following the rules of a 3 + 3 design (dose de-escalation rules: if patients treated at dose level 1 show signs of DLT, dose of instilled rBCG will be reduced to level -1, which is 10 times lower than level 1).

The dose levels were as follows:
- Dose level 1: 1 - 19.2 x 10⁸ CFUs of rBCG
- Dose level -1: 1 - 19.2 x 10⁷ CFUs of rBCG

### 1.4 Selection of patients

### 1.4.1 Inclusion criteria

- Histologically confirmed diagnosis of recurrent NMIBC including repeat TURB confirming tumor-free state of the bladder; for patients with pure CIS no repeat TURB is necessary.
- Negative cytology before start of treatment except for patients with concomitant CIS.
- Planned treatment start is 2-6 weeks after last TURB.
- One previous cycle of intravesical BCG (induction phase with at least 5 instillations ± maintenance) not more than 5 years ago for NMIBC
- Recurrent high-grade NMIBC for progression (progression score 7-23) based on the European Organization for Research and Treatment of Cancer scoring system, failing BCG therapy (Babjuk M, Eur Urol 2008 PMID 18468779), for whom radical cystectomy or re-induction with standard BCG is indicated.

This particularly includes patients with high grade NMBIC tumor(s) present at both 3 and 6 months after start of the previous cycle of BCG therapy, and any worsening of the disease under BCG treatment such as higher number of recurrences, higher tumor category or higher grade, or appearances of CIS, including low grade tumors, in spite of an initial response.

### 1.3.2 Exclusion criteria

- Current or previous ≥ T2 urothelial carcinoma (UC) of the urinary bladder.
- Concomitant UC of upper urinary tract, prostate or urethra, small cell carcinoma of the bladder, micropapillary urothelial carcinoma, pure squamous cell carcinoma of the bladder, pure adenocarcinoma, lympho-vascular invasion in the TURB specimen..
- Previous or current evidence of UC in a bladder diverticulum.
- Evidence of metastatic disease in the (thoraco-) abdominal CT scan at registration.
- Bladder surgery or dramatic catheterization or TURB within 2 weeks prior to the start of treatment.
- Administration of systemic cytotoxic agents within the past 3 years or administration of repeated cytostatic/cytotoxic drugs by intravesical instillations after EUC recurrence after the first BCG therapy (during the past 5 years).

### 1.4 Current status

Clinical phase I is completed.

Phase II was conducted with dose level 1: 1-19.2 x 10⁸ CFU of rBCG.

The active agent was administered in 50 ml drug solution containing dextran, glucose, 0.9% sodium chloride, 0.025% Tween 80 and water for injection.

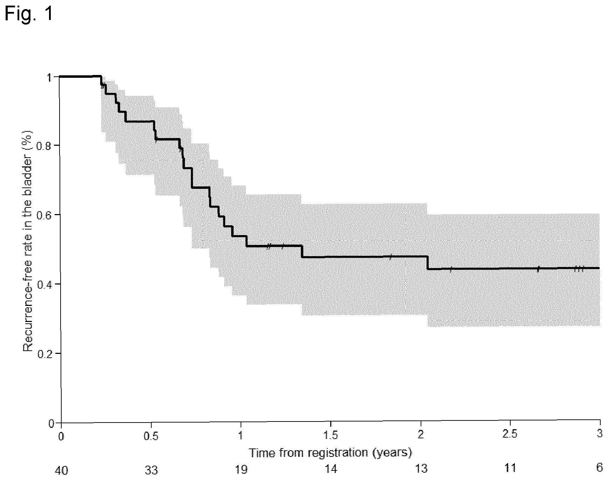

40 patients were included, all with high-grade tumors as specified above. The rate of CIS was high (68%). Recurrent-free state in the bladder (recurrence-free survival at 60 weeks was 49.3% and at three years 43.7 % with a 95% confidence interval [26,9%, 59,4%] as shown in Fig. 1.

The follow-up data for recurrence in the bladder status of all evaluable phase II patients at 3 years are shown below. 20 patients experienced a confirmed event and 1 patient an unconfirmed event. Ten patients remained alive and on follow-up for this endpoint.

| **Variable** | **Full Analysis Set (N=40) n** |
|---|---|
| Recurrence in the bladder status | |
| • Recurrence in the bladder | 20 |
| • Recurrence in the bladder (unconfirmed) | 1 |
| • Censored at last assessment (alive) | 10 |
| • Censored at last assessment (dead) | 1 |
| • Censored at last assessment (lost to follow-up) | 4 |
| • Censored at start of new treatment | 2 |
| • Censored at cystectomy date | 2 |

The treatment was safe and well tolerated, with only 5% of patients unable to tolerate adequate induction therapy. No dose-limiting toxicity occurred and no grade 3 or 4 adverse events were observed.

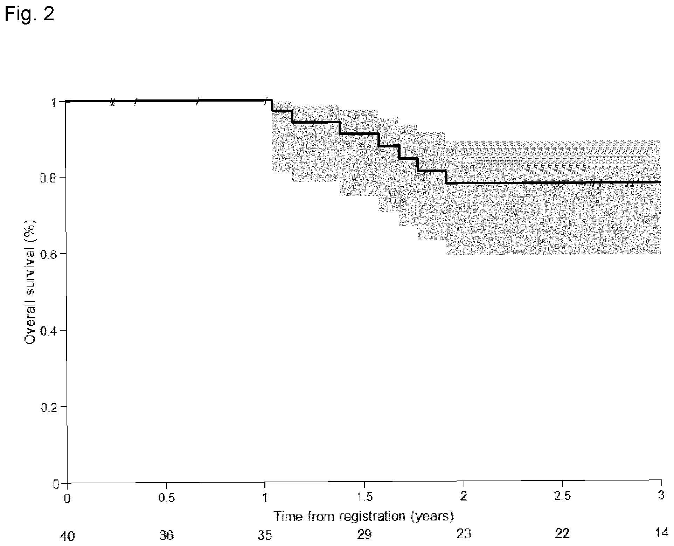

Overall survival after a median follow-up time of 2.9 years is shown in Fig. 2

## Claims

1. A recombinant Mycobacterium cell, which comprises a recombinant nucleic acid molecule encoding a fusion polypeptide comprising:
(a) a domain capable of eliciting an immune response, and
(b) a phagolysosomal escape domain
for use in a method, wherein the recombinant Mycobacterium cell is administered as an immunotherapeutic agent to a subject, e.g. a human subject suffering from bladder carcinoma, particularly from non-muscle-invasive bladder carcinoma as a second-line therapy.

2. The cell of claim 1 for the use of claim 1, which is a urease-deficient, recombinant *M. bovis* BCG cell from strain Danish subtype Prague and the domain capable of eliciting an immune response is selected from immunogenic peptides or polypeptides from *M. bovis* or *M. tuberculosis.*

3. The cell of claim 1 or 2 for the use of claim 1, wherein the recombinant nucleic acid molecule does not comprise any functional selection marker.

4. The cell of any one of claims 1-3 for the use of claim 1, wherein the fusion polypeptide comprises
(a) a domain capable of eliciting an immune response comprising the amino acid sequence from aa.41 to aa.51 in SEQ ID No.2, and
(b) a Listeria phagolysosomal escape domain encoded by a nucleic acid molecule selected from
(i) a nucleotide sequence comprising nucleotides 211-1722 as shown in SEQ ID No.1,
(ii) a nucleotide sequence which encodes the same amino acid sequence as the sequence from (i), and
(iii) a nucleotide sequence hybridizing under stringent conditions with the sequence from (i) or (ii).

5. The cell of any one of claims 1-4 for the use of claim 1, wherein the administration comprises vesicular instillation into the urinary bladder.

6. The cell of any one of claims 1-4 for the use of claim 1 or 5 in the treatment of a subject having previously undergone at least one unsuccessful therapy with recurrent bladder carcinoma with standard BCG.

7. The cell of any one of claims 1-4 for the use of any one of claims 1, 5 or 6, wherein the immunotherapeutic agent is administered into the bladder according to a schedule involving weekly instillations during (i) an induction phase with e.g. 6 weekly instillations, a first maintenance phase after about 3 months with e.g. 3 weekly instillations, a second maintenance phase after about 6 months with e.g. 3 instillations and a third maintenance phase after about 12 months with e.g. 3 instillations.

8. The cell of any one of claims 1-4 for the use of any one of claims 1 or 5-7, wherein the immunotherapeutic agent is administered at a dose of from about 10⁷ to 2 x 10⁸ CFU per administration.

9. The cell of any one of claims 1-4 for the use of any one of claims 1 or 5-8 wherein disease recurrence is inhibited for a time period of at least 1 year, particularly for a time period of at least 2 years or of at least 3 years.

10. The cell of any one of claims 1-4 for the use of any one of claims 1 or 5-9 wherein the immunotherapy provides a disease free time period of at least 1 year, particularly of at least 2 years or of at least 3 years for at least 30% of the patients and particularly for at least 45% of the patients.

11. The cell of any one of claims 1-4 for the use of any one of claims 1 or 5-10 wherein disease recurrence is blocked for at least one further year in a subject without disease recurrence for a time period of 2 years after treatment start.

12. The cell of any one of claims 1-4 for the use of any one of claims 1 or 5-10 wherein overall survival is increased in a time period of at least 1 year, particularly in a time period of at least 2 years or of at least 3 years.

13. The cell of any one of claims 1-4 for the use of any one of claims 1 or 5-12 wherein the need of cystectomy is reduced or avoided, e.g. for a time period of at least a 1 year, particularly for a time period of at least 2 years or of at least 3 years in at least 30% of the patients and particularly for at least 45% of the patients.

14. A method for the immunotherapeutic treatment of bladder carcinoma, particularly of non-muscle-invasive bladder carcinoma in a subject, particularly in a human subject, comprising administering to said subject as a second-line therapy a recombinant Mycobacterium cell, which comprises a recombinant nucleic acid molecule encoding a fusion polypeptide comprising:
(a) a domain capable of eliciting an immune response, and
(b) a phagolysosomal escape domain.
